# EUROPÄISCHE PATENTSCHRIFT

(11) **EP 0 934 258 B1**
(45) Veröffentlichungstag und Bekanntmachung des Hinweises auf die Patenterteilung: **29.08.2001**
(21) Anmeldenummer: 97921762.7
(22) Anmeldetag: 25.04.1997
(51) Int. Cl.: C07C 271/28, A01N 47/24, C07C 327/58

(54) **PHENYLCARBAMATE, VERFAHREN ZU IHRER HERSTELLUNG UND SIE ENTHALTENDE MITTEL**
PHENYLCARBAMATES, METHODS FOR PREPARING THEM AND AGENTS CONTAINING THEM
PHENYLCARBAMATES, LEURS PROCEDES DE PREPARATION ET AGENTS LES CONTENANT

(30) Priorität: 10.05.1996 DE 19618852
(43) Veröffentlichungstag der Anmeldung: 11.08.1999
(73) Patentinhaber: BASF AKTIENGESELLSCHAFT, 67056 Ludwigshafen (DE)
(72) Erfinder: BAYER, Herbert, D-68159 Mannheim (DE); MÜLLER, Bernd, D-67227 Frankenthal (DE); MÜLLER, Ruth, D-67159 Friedelsheim (DE); SAUTER, Hubert, D-68167 Mannheim (DE); GROTE, Thomas, D-67105 Schifferstadt (DE); GRAMMENOS, Wassilios, D-67063 Ludwigshafen (DE); KIRSTGEN, Reinhard, D-67434 Neustadt (DE); OBERDORF, Klaus, D-69117 Heidelberg (DE); PTOCK, Arne, D-67067 Ludwigshafen (DE); GÖTZ, Norbert, D-67547 Worms (DE); RACK, Michael, D-69123 Heidelberg (DE); RÖHL, Franz, D-67105 Schifferstadt (DE); AMMERMANN, Eberhard, D-64646 Heppenheim (DE); HARRIES, Volker, D-67227 Frankenthal (DE); LORENZ, Gisela, D-67434 Hambach (DE); STRATHMANN, Siegfried, D-67117 Limburgerhof (DE)
(74) Vertreter: Fitzner, Ulrich, Dr.
(86) Internationale Anmeldenummer: EP9702126
(87) Internationale Veröffentlichungsnummer: WO9743252

(56) Entgegenhaltungen:
- EP-A- 0 619 301
- WO-A-93/15046
- WO-A-96/16030

## Beschreibung

Die vorliegende Erfindung betrifft Phenylcarbamate der Formel I 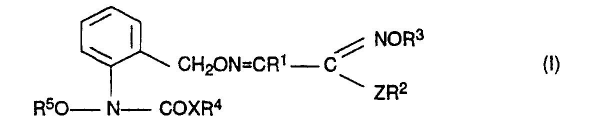 in der die Substituenten die folgende Bedeutung haben:
- X: eine direkte Bindung, O oder NR^{a};
R^{a} Wasserstoff oder Alkyl;
- Z: O, S oder NR^{b};
R^{b} Wasserstoff, C₁-C₆-Alkyl;, C₃-C₆-Alkenyl oder C₃-C₆-Alkinyl;
- R¹: Wasserstoff, C₁-C₆-Alkyl oder C₁-C₄-Halogenalkyl;
- R²: C₂-C₆-Alkyl, C₃-C₆-Alkenyl oder C₃-C₆-Alkinyl, wobei diese Gruppen partiell oder vollständig halogeniert sein können und/oder einen bis drei der folgenden Reste tragen können:
- Cyano, Nitro, Amino, Hydroxy,
- C₁-C₆-Alkoxy, C₁-C₆-Halogenalkoxy, C₁-C₆-Alkylthio, C₁-C₆-Halogenalkylthio, C₁-C₆-Alkylsulfonyl, C₁-C₆-Alkylsulfoxyl, C₁-C₆-Alkylamino, Di-C₁-C₆-alkylamino,
- C₃-C₆-Cycloalkyl, Aryl, Hetaryl oder Oxiranyl, wobei die cyclischen Reste ihrerseits partiell oder vollständig halogeniert sein können und/oder eine bis drei der folgenden Gruppen tragen können: Cyano, Nitro, Amino, Aminocarbonyl, Aminothiocarbonyl,_C₁-C₄-Alkyl, C₁-C₄-Halogenalkyl, C₁-C₄-Alkylcarbonyl, C₁-C₆-Alkoxy, C₁-C₆-Halogenalkoxy, C₁-C₆-Alkoxycarbonyl, C₁-C₆-Alkylthio, C₁-C₆-Halogenalkylthio, C₁-C₆-Alkylsulfonyl, C₁-C₆-Alkyl - sulfoxyl, C₁-C₆-Alkylamino, Di-C₁-C₆-alkylamino, C₁-C₆-Alkylaminocarbonyl, Di-C₁-C₆-alkylaminocarbonyl, C₂-C₆-Alkenyloxy und Phenyl;
Methyl, welches ein bis drei Halogenatome trägt und/oder einen der folgenden Reste trägt:
- Cyano, Nitro,
- C₁-C₆-Alkoxy, C₁-C₆-Halogenalkoxy, C₁-C₆-Alkylthio, C₁-C₆-Halogenalkylthio, C₁-C₆-Alkylsulfonyl, C₁-C₆-Alkylsulfoxyl, C₁-C₆-Alkylamino, Di-C₁-C₆-alkylamino,
- C₃-C₆-Cycloalkyl, Aryl, Hetaryl oder Oxiranyl, wobei die cyclischen Reste ihrerseits partiell oder vollständig halogeniert sein können und/oder eine bis drei der folgenden Gruppen tragen können: Cyano, Nitro, Amino, Aminocarbonyl, Aminothiocarbonyl,_C₁-C₄-Alkyl, C₁-C₄-Halogenalkyl, C₁-C₄-Alkylcarbonyl, C₁-C₆-Alkoxy, C₁-C₆-Halogenalkoxy, C₁-C₆-Alkoxycarbonyl, C₁-C₆-Alkylthio, C₁-C₆-Halogenalkylthio, C₁-C₆-Alkylsulfonyl, C₁-C₆-Alkylsulfoxyl, C₁-C₆-Alkylamino, Di-C₁-C₆-alkylamino, C₁-C₆-Alkylaminocarbonyl, Di-C₁-C₆-alkylaminocarbonyl, C₂-C₆-Alkenyloxy und Phenyl;
C₃-C₆-Cycloalkyl, wobei diese Gruppen partiell oder vollständig halogeniert sein können und/oder einen bis drei der folgenden Reste tragen können:
- Cyano, Nitro, Amino, Hydroxy,
- C₁-C₄-Alkyl, C₁-C₄-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfonyl, C₁-C₆-Alkylsulfoxyl, C₁-C₆-Alkylamino, Di-C₁-C₆-alkylamino,
- R³: C₁-C₆-Alkyl, C₂-C₆-Alkenyl oder C₂-C₆-Alkinyl, wobei diese Gruppen partiell oder vollständig halogeniert sein können und/oder einen bis drei der folgenden Reste tragen können:
- Cyano, C₁-C₃-Alkoxy,
- Oxiranyl oder C₃-C₆-Cycloalkyl, wobei die cyclischen Reste ihrerseits partiell oder vollständig halogeniert sein können und/oder eine bis drei C₁-C₄-Alkylgruppen tragen können;
- R⁴: Alkyl, Alkenyl oder Alkinyl, und für den Fall, daß X für NR^{a} steht, zusätzlich Wasserstoff;
- R⁵: Wasserstoff,
ggf. subst. Alkyl, Alkenyl, Alkinyl, Alkylcarbonyl oder Alkoxycarbonyl.

Außerdem betrifft die Erfindung Verfahren zur Herstellung dieser Verbindungen sowie sie enthaltende Mittel zur Bekämpfung von tierischen Schädlingen und Schadpilzen.

Aus der Literatur sind Anilide zur Bekämpfung von tierischen Schädlingen und Schadpilzen bekannt (WO-A 93/15,046; DE Anm. Nr. P 44 41 674.1).

Demgegenüber lagen der vorliegenden Erfindung Verbindungen mit verbesserter Wirkung als Aufgabe zugrunde.

Demgemäß wurden sie eingangs definierten Phenylcarbamate I gefunden. Außerdem wurden Verfahren zu ihrer Herstellung sowie sie enthaltende Mittel zur Bekämpfung von tierischen Schädlingen und Schadpilzen gefunden.

Die Verbindungen I sind auf verschiedenen Wegen nach an sich in der Literatur bekannten Verfahren erhältlich. Grundsätzlich ist es bei der Synthese der Verbindungen I unerheblich, ob zunächst die Gruppierung N(OR⁵)-COXR⁴ oder die Gruppe -CH₂ON=CR¹-C(ZR²)=NOR³ aufgebaut wird. Die verschiedenen Methoden zum Aufbau der N(OR⁵)-COXR⁴-Gruppierung (in den nachfolgenden Formeln z.T. mit dem Zeichen # verkürzt dargestellt) sind beispielsweise aus der eingangs zitierten Literatur bekannt.

Beim Aufbau der Gruppe -CH₂ON=CR¹-C(ZR²)=NOR³ geht man im allgemeinen so vor, daß man ein ein Benzylderivat der Formel II mit einem Hydroxyimin der Formel III umsetzt.

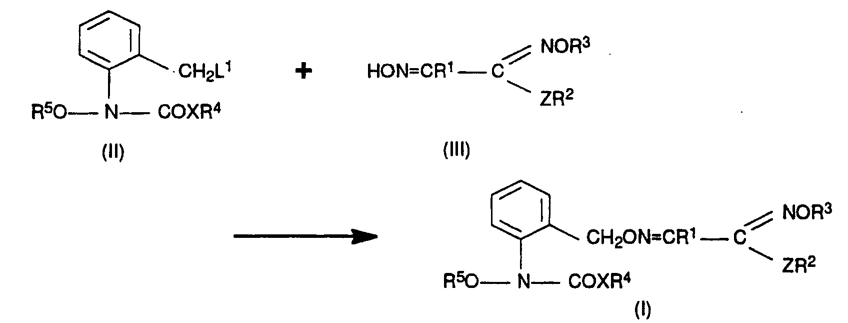

L¹ in der Formel II steht für eine nucleophil austauschbare Abgangsgruppe, z.B. Halogen oder Sulfonatgruppen, vorzugsweise Chlor, Brom, Iod, Mesylat, Tosylat und Triflat.

Die Umsetzung erfolgt in an sich bekannter Weise in eienm inerten organischen Lösungsmittel in Gegenwart einer Base (z.B. Natriumhydrid, Kaliumhydroxid, Kaliumcarbonat und Triethylamin) gemäß den in Houben-Weyl, Bd. E 14b, S. 370f und Houben-Weyl, Bd. 10/1, S. 1189f beschriebenen Methoden.

Das benötigte Hydroxyimin III erhält man beispielsweise durch Umsetzung eines entsprechenden Dihydroxyimins IV mit einem nucleophil substituierten Reagens VIa.

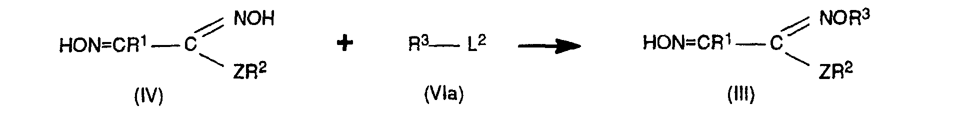

L² in der Formel VIa steht für eine nucleophil austauschbare Abgangsgruppe, z.B. Halogen oder Sulfonatgruppen, vorzugsweise Chlor, Brom, Iod, Mesylat, Tosylat und Triflat.

Die Umsetzung erfolgt in an sich bekannter Weise in einem inerten organischen Lösungsmittel in Gegenwart einer Base (z.B. Kaliumcarbonat, Kaliumhydroxid, Natriumhydrid, Pyridin und Triethylamin) gemäß den in Houben-Weyl, Bd. E 14b, S. 307f, S. 370f und S. 385f; Houben-Weyl, Bd. 10/4, S. 55f, S. 180f und S. 217f; Houben-Weyl, Bd. E 5, S. 780f beschriebenen Methoden.

Die Verbindungen IV sind bekannt (DE-A 26 21 102) oder können nach bekannter Methoden hergestellt werden.

Alternativ können die Verbindungen I auch dadurch erhalten werden, daß man das Benzylderivat II zunächst mit einem Dihydroxyinim IV in ein entsprechendes Benzyloxim V uberführt und V anschließend mit dem nucleophil substituierten Reagens VIa zu I umsetzt.

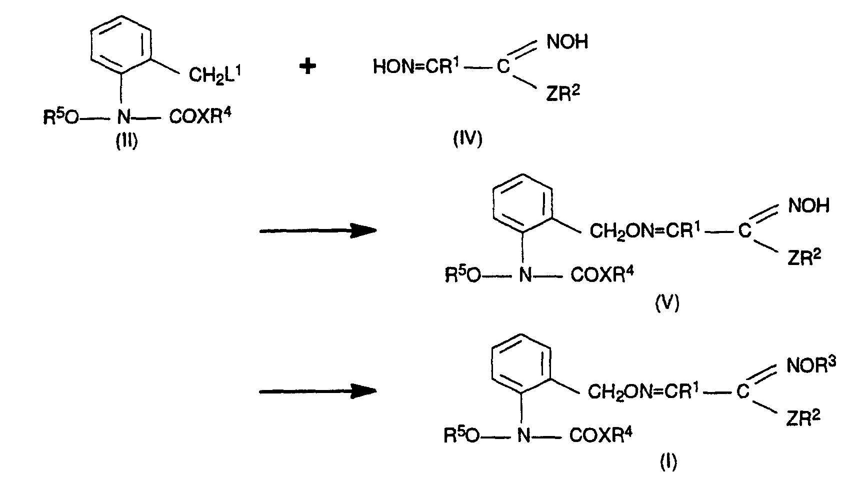

Die Umsetzung erfolgt in an sich bekannter Weise in einem inerten organischen Lösungsmittel in Gegenwart einer Base (z.B. Kaliumcarbonat, Kaliumhydroxid, Natriumhydrid, Pyridin und Triethylamin) gemäß den in Houben-Weyl, Bd. 10/1, S. 1189f; Houben-Weyl, Bd. E 14b, S. 307f, S. 370f und S. 385f; Houben-Weyl, Bd. 10/4, S. 55f, S. 180f und S. 217f; Houben-Weyl, Bd. E 5, S. 780f beschriebenen Methoden.

Nach einem weiteren Verfahren können die Verbindungen I auch dadurch erhalten werden, daß man das Benzylderivat II zunächst mit einem Hydroxyiminoester der Formel IVa in den Benzyloxyiminoester Va überführt und Va zur entsprechenden Säure Vb verseift. Durch Umsetzung der Säure Vb mit einem Hydroxylamin der Formel IXa oder dessen Salz IXb erhält man die entsprechende Hydroxamsäure Xa, die mit üblichen Halogenierungsmitteln in die entsprechenden Hydroxamsäurehalogenide Xb überführt werden können. Aus den Hydroxamsäurehalogeniden Xb erhält man durch Umsetzung mit nucleophilen Verbindungen XI die Verbindungen I.

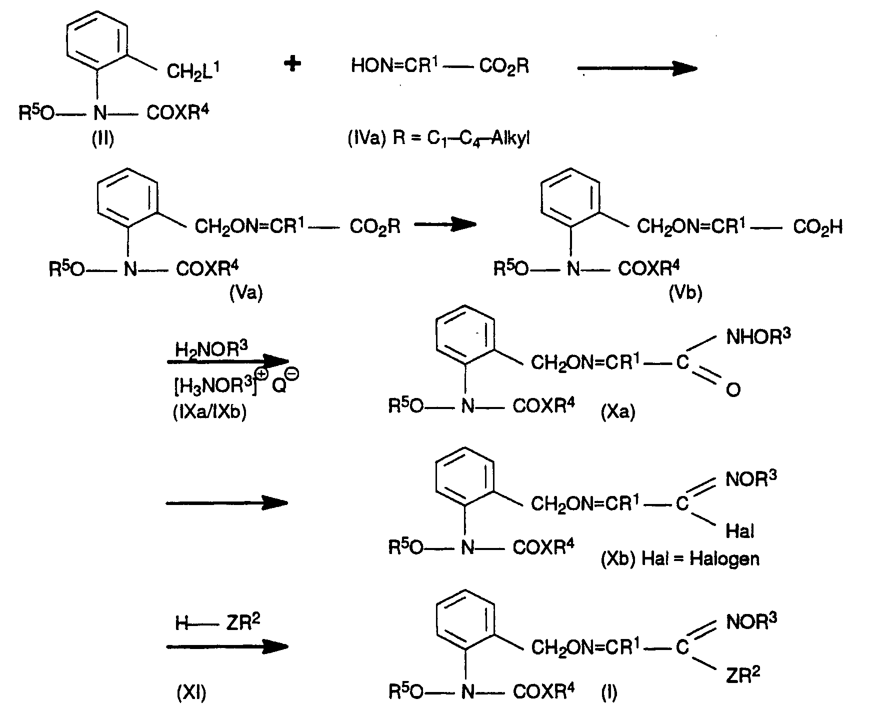

R in den Formeln IVa und Va steht für eine C₁-C₄-Alkylgruppe (insbesondere Methyl, Ethyl, Isopropyl und tert.-Butyl); Q⁻ in der Formel IXb bedeutet das Anion einer anorganischen Säure (insbesondere ein Halogenid wie Chlorid); Hal in der Formel Xb steht für ein Halogenatom wie Chlor, Brom und Iod.

Die Umsetzung des Benzylderivats II mit dem Hydroxyiminoester IVa erfolgt im wie im allgemeinen und im besonderen für die Umsetzung von II mit III beschrieben.

Die Verbindungen IVa sind bekannt [J. Am. Chem. Soc. 100, 1857 (1978); Bull. Soc. Chim. Fr. 1975, 252; Bull. Soc. Chim. Fr. 31, 1054 (1904); US-A 3,584,032] oder können nach den dort beschriebenen Methoden erhalten werden.

Die Verseifung des Esters Va zu Vb erfolgt analog literaturbekannten Methoden (vgl. Houben-Weyl Bd. E 5, S. 223f) bei Temperaturen von 0°C bis 150°C, vorzugsweise 20°C bis 80°C in Gegenwart einer Säure oder Base.

Geeignete Lösungsmittel sind Wasser, aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Wasser, Methanol und Ethanol.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetallcarbonate wie Lithiumcarbonat und Calziumcarbonat sowie Alkalimetallhydrogencarbonate wie Natriumhydrogencarbonat, in Betracht.

Besonders bevorzugt werden NaOH und KOH.

Die Basen werden im allgemeinen äquimolar oder im Überschuß eingesetzt.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propion-säure, Oxalsäure, Zitronensäure und Trifluoressigsäure Verwendung.

Die Säuren werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt.

Die Umsetzung der Säure Vb mit einem Hydroxylamin der Formel IXa oder dessen Salz IXb erfolgt üblicherweise bei Temperaturen von -10°C bis 120°C, vorzugsweise 0°C bis 50°C, in Gegenwart von Aktivierungsreagentien gemäß den in Houben-Weyl Bd. E 5, S. 1141f beschriebenen Methoden.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Tetrahydrofuran.

Es können auch Gemische der genannten Lösungsmittel verwendet werden.

Als Aktivierungsreagentien eignen sich Säurehalogenidbildner wie Phosgen, Phosphortribromid, Phosphortrichlorid, Phosphorpentachlorid, Phosphoroxychlorid, Thionylchlorid oder Oxalylchlorid; Anhydridbildner wie Chlorameisensäureethylester oder Methansulfonylchlorid; Carbodiimide wie N,N'-Dicyclohexylcarbodiimid oder andere übliche Mittel wie N,N'-Carbonyldiimidazol oder Triphenylphosphin in Tetrachlorkohlenstoff.

Besonders bevorzugt werden Thionylchlorid, Oxalylchlorid, N,N'-Carbonyldiimidazol.

Die Aktivierungsreagentien werden im allgemeinen äquimolar oder im Überschuß verwendet.

Bei der anschließenden Halogenierung der Hydroxamsäure Xa zu den Halogeniden Xb verwendet man übliche Halogenierungsmittel (z.B.Thionylchlorid, Oxalylchlorid, Phosphortribromid, Phosphortrichlorid, Phosphorpentachlorid, Phosphoroxychlorid, Triphenylphosphin und Tetrachlorkohlenstoff bzw. Tetrabromkohlenstoff); die Umsetzung erfolgt üblicherweise bei Temperaturen von -20°C bis 100°C, vorzugsweise -10°C bis 80°C gemäß bekannten Verfahren [Houben-Weyl Bd. E5, S. 631; J. Org. Chem. 36, 233 (1971); J. Org. Chem. 57, 3245 (1992)].

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Acetonitril, Toluol und Tetrahydrofuran.

Es können auch Gemische der genannten Lösungsmittel verwendet werden.

Die Edukte werden im allgemeinen in äquimolaren Mengen miteinander umgesetzt. Es kann für die Ausbeute vorteilhaft sein, das Halogenierungsmittel in einem Über- oder Unterschuß bezogen auf Xa einzusetzen.

Aus den so erhaltenen Hydroxamsäurehalogeniden Xb erhält man die Verbindungen I durch Umsetzung mit nucleophilen Verbindungen XI gemäß allgemein bekannten Verfahren [Houben-Weyl Bd. E 5, S. 826f und S. 1280f; J. Org. Chem. 36, 233 (1971); Collect. Czech. Chem. Commun. 48, 596 (1983); J. Org. Chem. 46, 3623 (1981)], üblicherweise bei Temperaturen von 0°C bis 150°C, vorzugsweise 20°C bis 100°C in Gegenwart einer Base.

Geeignete Lösungsmittel sind Wasser, aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Dimethylformamid, Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydrid, Kaliumhydrid, Kaliumcarbonat, Triethylamin und Pyridin.

Die Basen werden im allgemeinen äquimolar, im Überschuß oder gegebenenfalls als Lösungsmittel verwendet.

Die Edukte werden im allgemeinen in äquimolaren Mengen miteinander umgesetzt. Es kann für die Ausbeute vorteilhaft sein, XI in einem Überschuß bezogen auf Xb einzusetzen.

Verbindungen I, in denen Z für Sauerstoff steht, erhält man besonders bevorzugt im Sinne des vorstehend beschriebenen Verfahrens dadurch, daß man die Hydroxamsäuren Xa direkt in Gegenwart einer Base in einem inerten organischen Lösungsmittel mit einer Verbindung VId umsetzt [vgl. Houben-Weyl Bd. E 5, S. 826f; Aust. J. Chem. 27, 1341 (1974)].

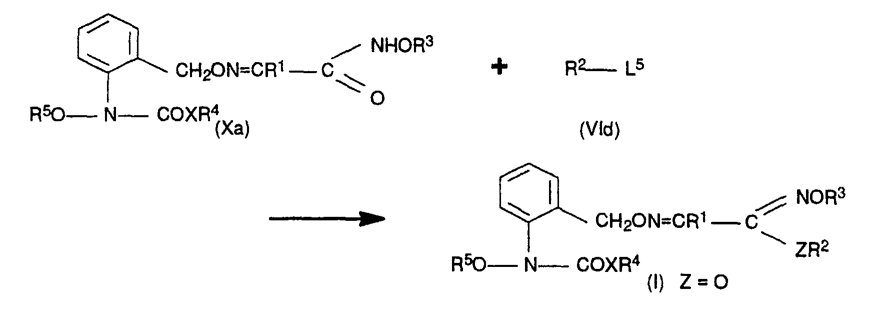

L⁵ in der Formel VId steht für eine nucleophil austauschbare Abgangsgruppe, z.B. Halogen oder Sulfonatgruppen, vorzugsweise Chlor, Brom, Iod, Mesylat, Tosylat und Triflat.

Diese Umsetzung erfolgt üblicherweise bei Temperaturen von 0°C bis 150°C, vorzugsweise 20°C bis 180°C.

Geeignete Lösungsmittel sind Wasser, aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Dimethylformamid und Tertrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Kaliumcarbonat, Kaliumhydroxyd, Natriumhydrid, Pyridin und Triethylamin.

Die Basen werden im allgemeinen in äquimolar, im Überschuß oder gegebenenfalls als Lösungsmittel verwendet.

Eine weitere Möglichkeit zur Herstellung der Verbindungen I ist die Umsetzung des Benzylderivats II mit N-Hydroxyphthalimid und nachfolgender Hydrazinolyse zum Benzylhydroxylamin IIa und die weitere Umsetzung von IIa mit einer Carbonylverbindung XII.

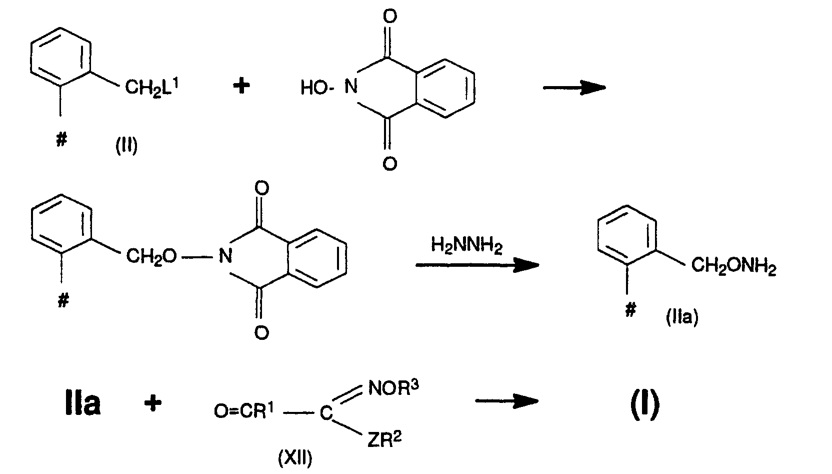

Die Umsetzung erfolgt in an sich bekannter Weise in einem inerten organischen Lösungsmittel gemäß den in EP-A 463 488 und EP-A 585 751 beschriebenen Methoden.

Die benötigten Carbonylverbindungen XII erhält man beispielsweise durch Umsetzung der entsprechenden Hydroxyiminocarbonylverbindungen XIIa mit einem nucleophil substituierten Reagens VIa.

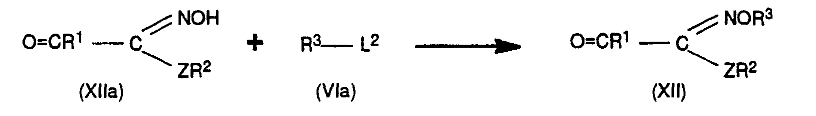

Die Umsetzung erfolgt in an sich bekannter Weise in einem inerten organischen Lösungsmittel gemäß den in Houben-Weyl Bd. 10/4, S. 217f, Houben-Weyl Bd. E 14b, S. 370f bzw. Heterocycles 36, 1027 (1993) beschriebenen Methoden.

Die Verbindungen XIIa sind bekannt (EP-A 056 161, EP-A 051 792, DE-A 21 11 459) oder können gemäß den in der Literatur beschriebenen Verfahren erhalten werden.

Entsprechend können die Verbindungen I auch dadurch erhalten werden, daß man das Benzylhydroxylamin IIa zunächst mit dem Hydroxyiminoderivat XIIa in das entsprechende Benzyloxyiminoderivat der Formel V überführt und V anschließend mit dem nucleophil substituierten Reagens VIa wie vorstehend beschrieben zu I umsetzt.

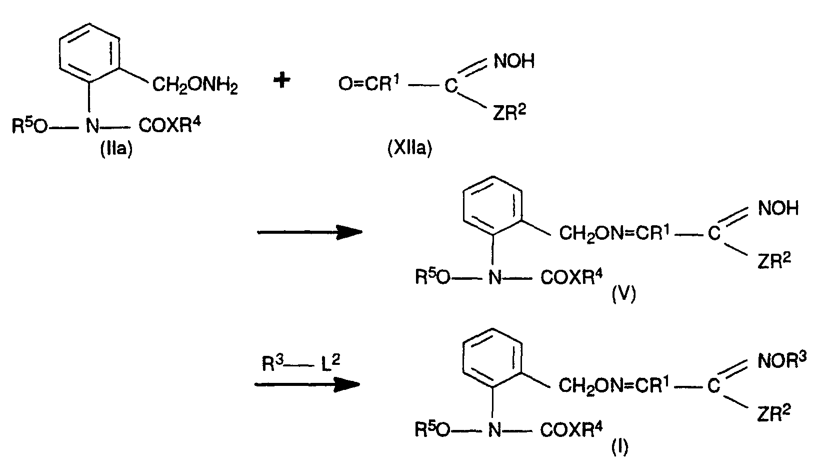

In Analogie zu den vorstehend beschriebenen Verfahren erhält man die Verbindungen Va bzw. Vb auch dadurch, daß man ein Benzylhydroxylamin der Formel IIa gemäß den vorstehend beschriebenen Bedingungen mit einem α-Ketosäureester XIIIa bzw. einer α-Ketosäure XIIIb umsetzt.

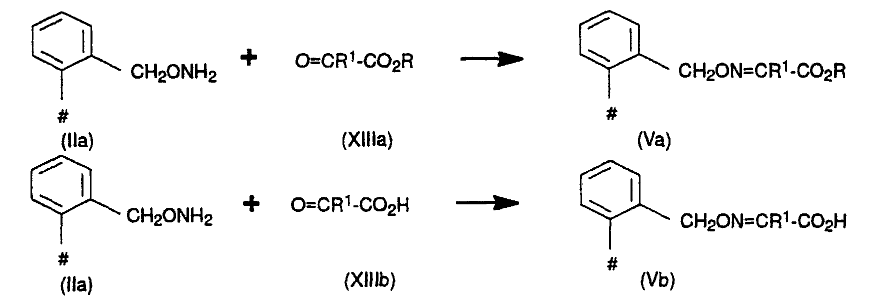

In entsprechender Weise können die Verbindungen Xa und Xb dadurch erhalten werden, daß man ein Benzylhydroxylaminoderivat der Formel IIa mit einer α-Ketohydroxamsäure XIVa bzw. einem α-Ketohydroxamsäurehalogenid XIVb umsetzt.

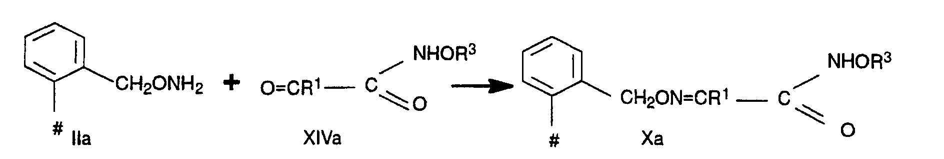

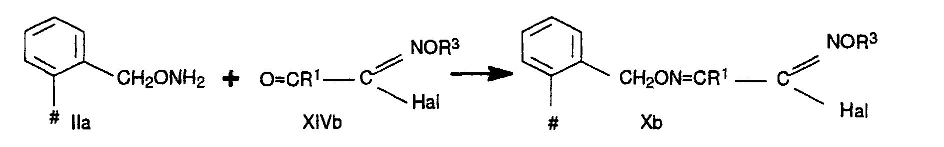

Die Verbindungen XIVb sind bekannt [Arch. Pharm. 310, 30f (1977)] oder können nach den in der Literatur beschriebenen Methoden hergestellt werden.

Der Aufbau der Gruppierung -N(OR⁵)-COXR⁴ ist beispielsweise aus der eingangs zitierten Literatur bekannt. Zur besseren Übersichtlichkeit ist in den folgenden Reaktionsschemata die CH₂ON=CR¹-C(ZR²)=NOR³ Seitenkette bzw. eine geeignete Vorstufe zu dieser Gruppe mit * verkürzt dargestellt.
1. Verbindungen I, in denen R⁵ Wasserstoff bedeutet (I.a), erhält man im allgemeinen dadurch, daß man ein Nitrobenzol der Formel VII zum entsprechenden Hydroxylamin VIII reduziert und VIII anschließend durch Umsetzung mit einem Acylierungsmittel der Formel VIb zu I.a umsetzt. 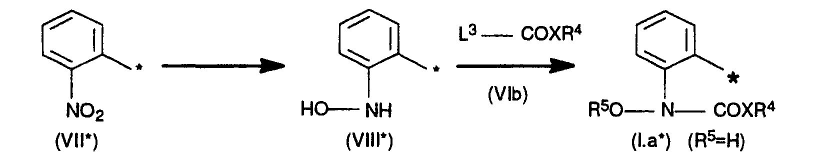 L³ in der Formel VIb steht beispielsweise für Halogen oder Aryloxy, insbesondere Chlor und Phenoxy.
   a) Die Reduktion von VII bzw. VII* zum Hydroxylamin VIII bzw. VIII* erfolgt üblicherweise bei Temperaturen von -30°C bis 80°C, vorzugsweise 0°C bis 60°C in einem inerten organischen Lösungsmittel in Gegenwart eines Katalysators *[vgl*. *DE Anm*. *Nr*. *19 50 27 00.0]*.
   b) Die Umsetzung von den Hydroxylamins VIII bzw. VIII* mit VIb erfolgt üblicherweise bei Temperaturen von -20°C bis 60°C, vorzugsweise 0°C bis 30°C in einem inerten organischen Lösungsmittel in Gegenwart einer Base *[vgl*. *WO-A 93/15046]*.

   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Cyclohexan, Toluol, Methylenchlorid, tert.-Butylmethylether und Wasser. Es können auch Gemische der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Kaliumcarbonat, Natriumhydroxyd und Triethylamin.
   Die Basen werden im allgemeinen in katalytischen Mengen eingesetzt, sie können aber auch äquimolar, im Überschuß oder gegebenenfalls als Lösungsmittel verwendet werden.
2. Die Verbindungen I, in denen R⁵ nicht Wasserstoff bedeutet (I.b), erhält man dadurch, daß man eine Verbindungen der Formel I.a in an sich bekannter Weise mit einer Verbindung der Formel VIc umsetzt. 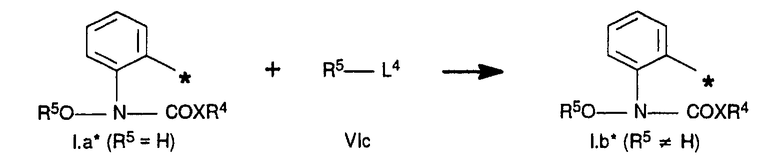 L⁴ in der Formel VIc bedeutet beispielsweise Halogen, Mesylat, Tosylat, Carboxylat und Sulfat, insbesondere Chlor, Brom, Mesylat und R²-OSO₃-.
   Diese Umsetzung erfolgt üblicherweise bei Temperaturen von -20°C bis 80°C, vorzugsweise 0°C bis 60°C in einem inerten organischen Lösungsmittel in Gegenwart einer Base *[vgl*. *WO-A 93/15*, *046]*.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Aceton, Toluol, tert.-Butylmethylether, Cyclohexan und Wasser. Es können auch Gemische der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Kaliumcarbonat, Natriumhydroxyd und Triethylamin.
   Die Basen werden im allgemeinen in katalytischen Mengen eingesetzt, sie können aber auch äquimolar, im Überschuß oder gegebenenfalls als Lösungsmittel verwendet werden.

In den vorstehenden Reaktionsschemata steht das Symbol * für die Seitenkette bzw. eine geeignete Vorstufe. Geeignete Vorstufen sind allgemein Verbindungen, in denen * für CH₃ oder CH₂-L¹ (≡ Formel II) steht.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Verbindungen II sind in der eingangs zitierten Literatur beschrieben oder können nach den in der Literatur beschriebenen Verfahren synthesisiert werden.

Die Verbindungen I können bei der bei der Herstellung aufgrund ihrer C=N-Doppelbindungen als E/Z-Isomerengemische anfallen, die z.B. durch Kristallisation oder Chromatographie in üblicher Weise in die Einzelverbindungen aufgetrennt werden können.

In Bezug auf die C=NOR³-Doppelbindung werden hinsichtlich ihrer Wirksamkeit die cis-Isomere der Verbindungen I bevorzugt (Konfiguration bezogen auf die OR³-Gruppe zur ZR²-Gruppe).

In Bezug auf die CH₂ON=CR¹-Doppelbindung werden hinsichtlich ihrer Wirksamkeit die *cis*-Isomere der Verbindungen I bevorzugt (Konfiguration bezogen auf die Reste R¹ im Verhältnis zur OCH₂-Gruppierung).

Sofern bei der Synthese Isomerengemische gebildet werden, ist die Trennung in die Einzelverbindungen im allgemeinen jedoch nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen, Pilzen und tierischen Schädlingen im behandelten Organismus erfolgen. Bei der eingangs angegebenen Definition der Verbindungen I wurden Sammelbegriffe verwendet, die allgemein repräsentativ für die folgenden Gruppen stehen:
Halogen: Fluor, Chlor, Brom und Jod;
Alkyl: gesättigte, geradkettige oder verzweigte Kohlenwasserstoffreste mit 1 bis 6 Kohlenstoffatomen wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Alkylamino: eine geradkettige oder verzweigte Alkylgruppe mit 1 bis 6 Kohlenstoffatomen (wie vorstehend genannt), welche über eine Aminogruppe (-NH-) an das Gerüst gebunden ist;
Dialkylamino: eine Aminogruppe, welche zwei voneinander unabhängige, geradkettige oder verzweigte Alkylgruppen mit jeweils 1 bis 6 Kohlenstoffatomen (wie vorstehend genannt) trägt;
Alkylaminocarbonyl: eine Alkylaminogruppe (wie vorstehend genannt), welche über eine Carbonylgruppe (-CO-) an das Gerüst gebunden ist;
Dialkylaminocarbonyl: eine Dialkylaminogruppe (wie vorstehend genannt), welche über eine Carbonylgruppe (-CO-) an das Gerüst gebunden ist;
Alkylcarbonyl: geradkettige oder verzweigte Alkylgruppen mit 1 bis 4 Kohlenstoffatomen (wie vorstehend genannt), welche über eine Carbonylgruppe (-CO-) an das Gerüst gebunden sind;
Alkylsulfonyl: geradkettige oder verzweigte Alkylgruppen mit 1 bis 6 Kohlenstoffatomen (wie vorstehend genannt), welche über eine Sulfonylgruppe (-SO₂-) an das Gerüst gebunden sind;
Alkylsulfoxyl: geradkettige oder verzweigte Alkylgruppen mit 1 bis 6 Kohlenstoffatomen (wie vorstehend genannt), welche über eine Sulfoxylgruppe [-S(O)-] an das Gerüst gebunden sind;
Halogenalkyl: geradkettige oder verzweigte Alkylgruppen mit 1 bis 4 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkylgruppen mit 1 bis 6 Kohlenstoffatomen (wie vorstehend genannt), welche über ein Sauerstoffatom (-O-) an das Gerüst gebunden sind;
Alkoxycarbonyl: geradkettige oder verzweigte Alkoxygruppen mit 1 bis 6 Kohlenstoffatomen (wie vorstehend genannt), welche über eine Carbonylgruppe (-CO-) an das Gerüst gebunden sind;
Halogenalkoxy: geradkettige oder verzweigte Halogenalkylgruppen mit 1 bis 6 Kohlenstoffatomen (wie vorstehend genannt), welche über ein Sauerstoffatom (-O-) an das Gerüst gebunden sind;
Alkylthio: geradkettige oder verzweigte Alkylgruppen mit 1 bis 6 Kohlenstoffatomen (wie vorstehend genannt), welche über ein Schwefelatom (-S-) an das Gerüst gebunden sind;
Halogenalkylthio: geradkettige oder verzweigte Halogenalkylgruppen mit 1 bis 6 Kohlenstoffatomen (wie vorstehend genannt), welche über ein Schwefelatom (-S-) an das Gerüst gebunden sind; Alkenyl: ungesättigte, geradkettige oder verzweigte Kohlenwasserstoffreste mit 2 bzw. 3 bis 6 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl. 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-l-butenyl, 1,2-Dimethyl-2-butenyl, 1,2-Dimethyl-3-butenyl, 1,3-Dimethyl-l-butenyl, 1,3-Dimethyl-2-butenyl, 1,3-Dimethyl-3-butenyl, 2,2-Dimethyl-3-butenyl, 2,3-Dimethyl-l-butenyl, 2,3-Dimethyl-2-butenyl, 2,3-Dimethyl-3-butenyl, 3,3-Dimethyl-l-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-l-methyl-2-propenyl, 1-Ethyl-2-methyl-1-propenyl und 1-Ethyl-2-methyl-2-propenyl;
Alkenyloxy: geradkettige oder verzweigte Alkenylgruppen mit 2 bis 6 Kohlenstoffatomen (wie vorstehend genannt), welche über ein Sauerstoffatom an das Gerüst gebunden sind;
Alkinyl: geradkettige oder verzweigte Kohlenwasserstoffgruppen mit 2 bis 6 Kohlenstoffatomen und einer Dreifachbindung in einer beliebigen Position wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl- 1-pentinyl, 3-Methyl-4-pentinyl, 4-Methyl-1-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-l-butinyl, 1-Ethyl-2-butinyl, 1-Ethyl-3-butinyl, 2-Ethyl-3-butinyl und 1-Ethyl-1-methyl-2-propinyl;
Cycloalkyl: monocyclische Alkylgruppen mit 3 bis 6 Kohlenstoffringgliedern wie Cyclopropyl, Cyclobutyl, Cyclopentyl und Cyclohexyl;
Aryl: ein aromatischer monocyclischer oder polycyclischer Kohlenwasserstoffrest wie Phenyl, Naphthyl und Anthracenyl;
Heteroaryl: ein aromatischer, monocyclischer oder polycyclischer Rest, welcher neben Kohlenstoff noch Heteroatome aus der Gruppe Sauerstoff, Schwefel und Stickstoff als Ringglieder enthalten kann, z.B.

- 5-gliedriges Heteroaryl, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
- 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Die Angabe *"partiell oder vollständig halogeniert"* soll zum Ausdruck bringen, daß in derart charakterisierten Gruppen die Wasserstoffatome (der Kohlenwasserstoffgruppen) zum Teil oder vollständig durch gleiche oder verschiedene Halogenatome wie vorstehend genannt ersetzt sein können.

Die Angabe "*gegebenfalls substituiert"* in Bewzug auf die bei R⁵ geannten Reste soll zum Ausdruck bringen, daß die Kohlenwasserstoff-Gruppen partiell oder vollständig halogeniert sein können und/oder einen bis drei (insbesondere einen) der folgenden Reste tragen können: Hydroxy, Cyano und C₁-C₄-Alkoxy.

Im Hinblick auf ihre biologische Wirkung sind insbesondere Verbindungen I bevorzugt, in denen X Sauerstoff bedeutet.

Gleichermaßen sind Verbindungen I bevorzugt, in denen X NH bedeutet.

Daneben sind Verbindungen I bevorzugt, in denen X eine direkte bindung bedeutet.

Ganz besonders bevorzugt sind Verbindungen I, in denen Z Sauerstoff bedeutet.

Gleichermaßen sind Verbindungen I bevorzugt, in denen R¹ C₁-C₄-Alkyl, insbesondere Methyl, bedeutet.

Daneben sind Verbindungen I bevorzugt, in denen R¹ C₁-C₄-Halogenalkyl, insbesondere Trifluormethyl, bedeutet.

Außerdem sind Verbindungen I bevorzugt, in denen Z Sauerstoff oder Schwefel bedeutet.

Desweiteren sind Verbindungen I bevorzugt, in denen Z NR^{b} bedeutet, wobei besonders Verbindungen I bevorzugt sind, in denen R^{b} für Wasserstoff oder C₁-C₄-Alkyl (insbesondere Wasserstoff oder Methyl) steht.

Gleichermaßen sind Verbindungen I bevorzugt, in denen R² für unsusbstituiertes oder substituiertes Benzyl oder für Alkyl, Alkenyl oder Alkinyl mit jeweils 2 bis 6 Kohlenstoffatomen steht.

Daneben sind Verbindungen I bevorzugt, in denen R² für Benzyl steht, welches partiell oder vollständig halogeniert sein kann und/oder einen bis drei der folgenden Reste tragen kann: Cyano, C₁-C₄-Alkyl, C₁-C₄-Halogenalkyl, C₁-C₄-Alkylcarbonyl, C₁-C₄-Alkoxy, C₁-C₄-Halogenalkoxy, C₁-C₄-Alkoxycarbonyl und Alkylthio.

Insbesondere sind Verbindungen I bevorzugt, in denen R² für C₂-C₆-Alkyl steht.

Außerdem sind Verbindungen I bevorzugt, in denen R² für C₃-C₆-Cycloalkyl steht.

Gleichermaßen sind Verbindungen I bevorzugt, in denen R³ für C₁-C₆-Alkyl (bevorzugt C₁-C₄-Alkyl, insbesondere C₁-C₂-Alkyl), welches partiell oder vollständig halogeniert sein kann, steht. Daneben sind Verbindungen I bevorzugt, in denen R³ für C₂-C₆-Alkenyl (bevorzugt C₃-C₆-Alkenyl, insbesondere C₃-C₄-Alkenyl), welches partiell oder vollständig halogeniert sein kann, steht.

Außerdem sind Verbindungen I bevorzugt, in denen R³ für C₂-C₆-Alkinyl (bevorzugt C₃-C₆-Alkinyl, insbesondere C₃-C₄-Alkinyl), welches partiell oder vollständig halogeniert sein kann, steht.

Insbesondere sind Verbindungen I bevorzugt, in denen R³ für Methyl steht.

Gleichermaßen sind Verbindungen I bevorzugt, in denen R⁴ für Methyl steht.

Desweiteren sind Verbindungen I bevorzugt, in denen R⁴ für Ethyl steht.

Gleichermaßen sind Verbindungen I bevorzugt, in denen R⁵ für Wasserstoff steht.

Desweiteren sind Verbindungen I bevorzugt, in denen R⁵ für Methyl steht.

Außerdem werden Verbindungen I bevorzugt, in denen R⁵ für Ethyl steht.

Daneben werden Verbindungen I bevorzugt, in denen R⁵ für Methoxymethyl, Allyl oder Propargyl steht.

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten, Deuteromyceten, Phycomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben,

Puccinia-Arten an Getreide, Rhizoctonia-Arten an Baumwolle, Reis und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Gemüse, Zierpflanzen, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der Verbindung I gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)disulfid;
Nitroderivate wie Dinitro-(1-methylheptyl)phenylcrotonat, 2-sec.-Butyl-4,6-dinitrophenyl-3,3-dimethylacrylat, 2-sec.-Butyl-4,6-dinitrophenyl-iso-propylcarbonat, 5-Nitro-iso-phthalsäure-di-iso-propylester;
heterocyclische Substanzen wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)phosphinyl]-3-phenyl-1,2,4-triazol, 2,3-Dicyano-1,4-dithioanthrachinon, 2-Thio-1,3-dithiolo-[4,5-b]-chinoxalin, 1-(Butylcarbamoyl)-2-benzimidazol-carbaminsäuremethylester, 2-Methoxycarbonylamino-benzimidazol, 2-(Furyl-(2))benzimidazol, 2-(Thiazolyl-(4))benzimidazol, N-(1,1,2,2-Tetrachlorethylthio)tetrahydrophthalimid, N-Trichlormethylthio-tetrahydrophthalimid, N-Trichlor-methylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlor-ethylacetal, Piperazin-1,4-diylbis-(1-(2,2,2-trichlor-ethyl)formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecylmorpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlor-phenoxyethyl)-N'-imidazol-yl-harnstoff, 1-(4-Chlorphenoxy)-3,3-dimethyl-1-(1H-1,2,4-triazol-1-yl)-2-butanon, (2-Chlorphenyl)-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)benzol, 1, 2-Bis-3-methoxycarbonyl-2-thioureido)benzol, [2-(4-Chlorphenyl)ethyl]-(1,1-dimethylethyl)-1H-1,2,4-triazol-1-ethanol, 1-[3-(2-Chlorphenyl)-1-(4-fluorphenyl)oxiran-2-yl-methyl]-lH-1,2,4-triazol sowie
verschiedene Fungizide wie Dodecylguanidinacetat, 3-[3-(3,5-Dimethyl-2-oxycyclohexyl)-2-hydroxyethyl)]glutarimid, Hexachlorbenzol, DL-Methyl-N-(2,6-dimethyl-phenyl)-N-furoyl(2)alaninat, DL-N-(2,6-Dimethyl-phenyl)-N-(2'-methoxyacetyl)alanin-methylester, N-(2,6-Dimethylphenyl)-N-chloracetyl-D,L-2-aminobutyrolacton, DL-N-(2,6-Dimethylphenyl)-N-(phenylacetyl)alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl)-1,3-oxazolidin-2,4-dion, 3-(3,5-Dichlorhenyl)-1-iso-propylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)pentyl]-1H-1,2,4-triazol, 2,4-Difluor-a-(1H-1,2,4-triazolyl-1-methyl)benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)methylsilyl)methyl)-1H-1,2,4-triazol.
Strobilurine wie Methyl-E-methoximino-[a-(o-tolyloxy)-o-to-lyl]acetat, Methyl-E-2-{2-[6-(2-cyanophenoxy)pyrimidin-4-yloxy]-phenyl}-3-methoxyacrylat, Methyl-E-methoximino-[a-(2-phenoxyphenyl)]acetamid, Methyl-E-methoximino-[a-(2,5-dimethylphenoxy)-o-tolyl]acetamid.
Anilinopyrimidine wie N-(4,6-Dimethylpyrimidin-2-yl)anilin, N-[4-Methyl-6-(1-propinyl)pyrimidin-2-yl]anilin, N-(4-Methyl-6-cyclopropyl-pyrimidin-2-yl)anilin.
Phenylpyrrole wie 4-(2,2-difluor-1,3-benzodioxol-4-yl)pyrrol-3-carbonitril.
Zimtsäureamide wie 3-(4-Chlorphenyl)-3-(3,4-dimethoxyphenyl)acrylsäuremorpholid.
(2RS,3SR)-1-[3-(2-Chlorphenyl)-2-[4-fluorphenyl]oxiran-2-ylmethyl]-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, FettalkoholethylenoxidKondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.% des Wirkstoffs enthält.
II. 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
V. 80 Gew.-Teile einer erfindungsgemäßen Verbindung werden mit 3 Gew.-Teilen des Natriumsalzes der Diisobutylnaphthalinalpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.%).
VII. 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
VIII.20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Angaben aufgeführt.

### Beispiel 1: Herstellung von (E)-N-Methoxy-N-{2-[(1'-methyl, 1'-methoxycarbonyl)iminooxymethyl]phenyl}carbaminsäure-methylester

Eine Mischung von 2,4 g (0,08 mol) Natriumhydrid (80%) in 50 ml Dimethylformamid wurde bei ca. 25°C mit einer Lösung von 8,5 g (0,073 mol) E-2-Hydroxyiminopropionsäure-methylester in 30 ml Dimethylformamid versetzt (Erwärmung auf 35°C). Die Mischung wurde auf ca. 25°C abgekühlt und anschließend mit einer Lösung aus 20 g (0,073 mol) N-(2-Brommethylphenyl)-N-methoxy-carbaminsäuremethylester in 50 ml Dimethylformamid versetzt. Nach 60 h bei ca. 25°C wurde das Reaktionsgemisch auf Wasser gegossen und mit tert.-Butyl-methylether extrahiert. Die organische Phase wurde mit Wasser gewaschen, getrocknet und bei vermindertem Druck eingeengt. Das so erhaltene Rohprodukt wurde säulenchromatographisch (Kieselgel, tert.-Butyl-methylether/n-Hexan) gereinigt. Man erhielt 15 g der Titelverbindung als farbloses Öl.
¹H-NMR (CDCl₃; δ in ppm): 2,10 (s, 3H); 3,73 (s, 3H); 3,77 (s, 3H); 3,83 (s, 3H); 5,35 (s, 2H); 7,32-7,51 (m,4H)

### Beispiel 2: Herstellung von (E)-N-Methoxy-N-{2-[(1'-methyl, 1'-hydroxycarbonyl)iminooxymethyl]phenyl)carbaminsäure-methylester

Eine Lösung von 15 g (48,4 mmol) der Verbindung aus Beispiel 1) in 30 ml Methanol wurde unter Eiskühlung mit 48 ml einer 1 m Kaliumhydroxid-Lösung versetzt. Nach 60 h bei ca. 25°C wurde das Reaktionsgemisch mit tert.-Butyl-methylether extrahiert. Die wäßrige Phase wurde mit verdünnter HCl angesäuert und anschließend mit tert.-Butyl-methylether extrahiert. Die so erhaltene organische Phase wurde getrocknet und bei vermindertem Druck eingeengt. Man erhielt so 9,5 g der Titelverbindung als hellgelbes Öl.
¹H-NMR (CDCl₃; δ in ppm): 2,07 (s, 3H); 3,74 (s, 3H); 3,83 (s, 3H); 5,29 (s, 2H); 7,33-7,52 (m, 4H)

### Beispiel 3: Herstellung von (E)-N-Methoxy-N-{2-[(1'-methyl, 1'-methoxyaminocarbonyl)iminooxymethyl]phenyl}carbaminsäure-methylester

Eine Lösung aus 9,0 g (30,4 mmol) der Verbindung aus Beispiel 2) in 80 ml Tetrahydrofuran wurde mit 4,9 g (30,4 mmol) Carbonyldiimidazol versetzt. Nach dem Ende der Gasentwicklung wurde die erhaltene Mischung mit 5,1 g (61 mmol) Methoxyammonium-hydrochlorid versetzt. Nach 16 h bei ca. 25°C wurde das Reaktionsgemisch auf Wasser gegossen und mit tert.-Butyl-methylether extrahiert. Die organische Phase wurde mit Wasser gewaschen, getrocknet und bei vermindertem Druck eingeengt. Man erhielt so 7,5 g der Titelverbindung als beige-farbenes Pulver. Fp.: 79-82°C
¹H-NMR (CDCl₃; δ in ppm) : 2,04 (s, 3H); 3,72 (s, 3H); 3,77 (s, 3H); 3,82 (s, 3H); 5,20 (s, 2H); 7,28-7,47 (m, 4H); 9,40 (s, br, 1H)

### Beispiel 4: Herstellung von (E,Z)-M-Methoxy-N-{2-[(1'-methyl, 1'-(1"-ethoxy, 1"-methoxyiminomethyl))iminooxymethyl]phenyl}carbaminsäure-methylester (Tabelle I, Nr. I.1)

Eine Mischung aus 1,3 g (4,4 mmol) der Verbindung aus Beispiel 3 und 1,2 g (8,8 mmol) Kaliumcarbonat in 50 ml Dimethylformamid wurde 20 min. bei ca. 25°C gerührt und anschließend mit 0,95 g (8,8 mmol) Bromethan versetzt. Nach 60 h bei ca. 25°C wurde das Reaktionsgemisch auf Wasser gegossen und mehrfach mit tert.-Butyl-methylether extrahiert. Die vereinigten organischen Phasen wurden mit Wasser gewaschen, getrocknet und bei vermindertem Druck eingeengt. Das so erhaltene Rohprodukt wurde säulenchromatographisch (Kieselgel, tert.-Butyl-methylether/n-Hexan) gereinigt. Man erhielt so 0,6 g der Titelverbindung als gelbliches Öl.
¹H-NMR (CDCl₃; δ in ppm): 1,24 (t, 3H); 2,06 (s, 3H); 3,74 (s, 3H); 3,79 (s, 3H); 3,88 (s, 3H); 4,14 (q, 2H); 5,27 (s, 2H); 7,32-7,52 (m, 4H)

### Beispiel 5: Herstellung von (E,Z)-N-Methoxy-N-{2-[(1'-methyl, 1'-(1"-brom, 1"-methoxyiminomethyl))iminooxymethyl]phenyl}carbaminsäuremethylester

Eine Mischung aus 10 g (31 mmol) der Verbindung aus Beispiel 3 und 40 g (0,15 mol) Triphenylphosphin in 350 ml Acetonitril wurde portionsweise mit 51 g (0,15 mol) Tetrabrommethan versetzt. Die so erhaltene Mischung wurde für 72 h refluxiert. Die Reaktionsmischung wurde bei ca. 25°C filtriert. Das Filtrat wurde eingeengt und der so erhaltene Rückstand säulenchromatographisch (Kieselgel; tert.-Butyl-methylether/Hexan) gereinigt. Man erhielt so 7 g der Titelverbindung als orangefarbenes Öl.
¹H-NMR (CDCl₃; δ in ppm): 2,17 (s, 3H); 3,74 (s, 3H); 3,78 (s, 3H); 4,08 (s, 3H); 5,30 (s, 2H); 7,32-7,88 (m, 4H)

### Beispiel 6: Herstellung von (E,Z)-N-Methoxy-N-Methoxy-N-{2-[(1'-methyl, 1'-(1"-ethylthio, 1"-methoxyiminomethyl))iminooxymethyl]phenyl)carbaminsäure-methylester (Tabelle I, Nr. I.19)

Zu einer Lösung von 1,5 g (3,9 mmol) der Verbindung aus Beispiel 5 in 50 ml Dimethylformamid gab man 0,7 g (8,6 mmol) Natriumethanthiolat und ließ 16 h bei Raumtemperatur nachrühren. Das Reaktionsgemisch wurde auf Wasser gegossen und mit Methyltert.-butylether extrahiert. Die organische Phase wurde mit Wasser gewaschen und eingeengt. Nach säulenchromatographischer Reinigung (Kieselgel; Methyl-tert.-butylether/Hexan) erhielt man 0,5 g der Titelverbindung als gelbes Öl.
¹H-NMR (CDCl₃; δ in ppm): 1,07 (t, 3H); 2,14 (s, 3H); 2,73 (q, 2H); 3,74 (s, 3H); 3,77 (s, 3H); 3,99 (s, 3H); 5,24 (s, 2H); 7,32-7,53 (m, 4H)

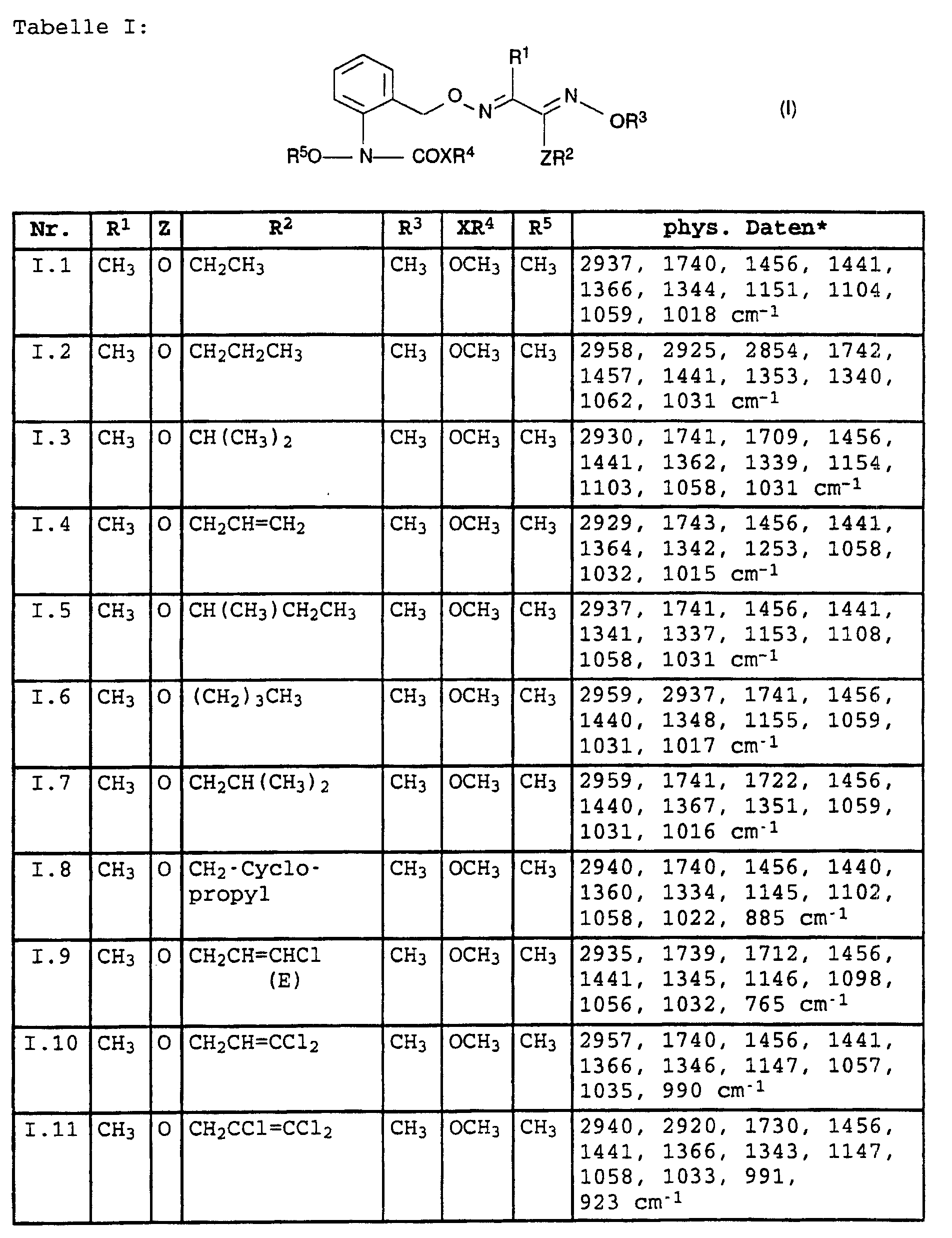

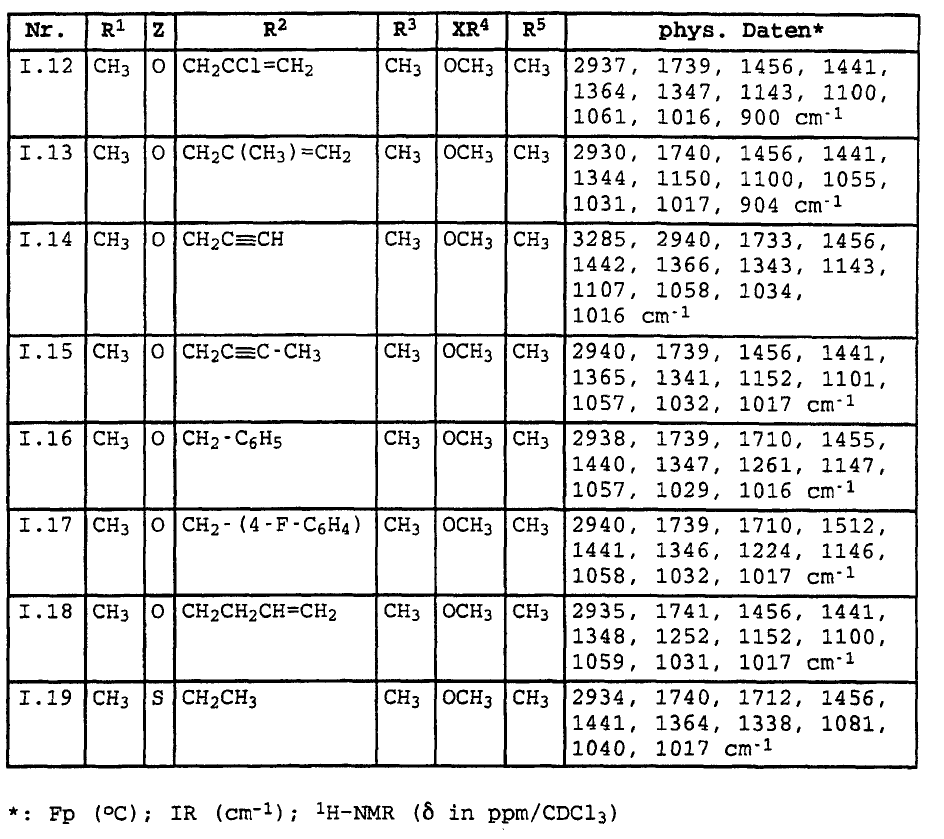

### Beispiele zur Wirkung gegen Schadpilze

Die fungizide Wirkung der Verbindungen der Formel I ließ sich durch folgende Versuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

Als Vergleichsverbindungen dienten die Verbindungen **A** (Verbindung Nr. 12, WO-A 96/16,030) und **B** (Verbindung Nr. 6, WO-A 96/16,030).

**Wirkung gegen Erysiphe graminis var.** tritici *(Weizenmehltau)* Blätter von Weizenkeimlingen (Sorte "Frühgold") wurden zunächst mit der wäßrigen Aufbereitung der Wirkstoffe behandelt (Aufwandmenge: 4 ppm). Nach ca. 24h wurden die Pflanzen mit Sporen des Weizenmehltaus (*Erysiphe graminis var. tritici*) bestäubt. Die so behandelten Pflanzen wurden anschließend für 7 Tage bei 20-22°C und einer relativen Luftfeuchtigkeit von 75-80% inkubiert. Anschließend wurde das Ausmaß der Pilzentwicklung ermittelt.

In diesem Test zeigten die mit den erfindungsgemäßen Verbindungen 1, 2, 3, 4, 5, 6, 7, 8 und 9 behandelten Pflanzen einen Befall von 15% und weniger. Die mit den Vergleichsverbindungen behandelten Pflanzen waren zu 40% (**A**) bzw. 65% (**B**) befallen, während die unbehandelten (Kontroll-) Pflanzen zu 80% befallen waren.

### Wirkung gegen Plasmopara viticola (Rebenperonospora)

Topfreben (Sorte: "Müller Thurgau") wurden mit der Wirkstoffaufbereitung tropfnaß gespritzt (Aufwandmenge: 250 ppm). Nach 8 Tagen wurden die Pflanzen mit einer Zoosporenaufschwemmung des Pilzes *Plasmopara viticola* besprüht und 5 Tage bei 20-30°C bei hoher Luftfeuchtigkeit aufbewahrt. Vor der Beurteilung wurden die Pflanzen danach für 16h bei hoher Luftfeuchtigkeit aufbewahrt. Die Auswertung erfolgte visuell.

In diesem Test zeigten die mit den erfindungsgemäßen Verbindungen 1, 2, 3, 4, 5, 6, 7, 8, 9, 10, 11, 12, 13, 14, 15, 16, 17, 18 und 19 behandelten Pflanzen einen Befall von 5% und weniger. Die mit der Vergleichsverbindung **A** behandelten Pflanzen waren zu 40% befallen, während die unbehandelten (Kontroll-) Pflanzen zu 70% befallen waren.

### Wirkung gegen Puccinia recondita (Weizenbraunrost)

Blätter von Weizensämlingen (Sorte "Kanzler") wurden mit Sporen des Braunrosts (*Puccinia recondita)* bestäubt. Die so behandelten Pflanzen wurden 24h bei 20-22°C und einer relativen Luftfeuchtigkeit von 90-95% inkubiert und anschließend mit der wäßrigen Wirkstoffaufbereitung behandelt (Aufwandmenge: 250 ppm). Nach weiteren 8 Tagen bei 20-22°C und 65-70% relativer Luftfeuchtigkeit wurde das Ausmaß der Pilzentwicklung ermittlelt. Die Auswertung erfolgte visuell.

In diesem Test zeigten die mit den erfindungsgemäßen Verbindungen 1, 2, 3, 4, 5, 6, 7, 8, 9, 10, 11, 12, 13, 14, 16, 17, 18 und 19 behandelten Pflanzen 0 bis 5 % Befall. Die mit der Vergleichsverbindung **B** behandelten Pflanzen waren zu 40% befallen, während die unbehandelten (Kontroll-) Pflanzen zu 70% befallen waren.

### Beispiele zur Wirkung gegen tierische Schädlinge

Die Wirkung der Verbindungen der allgemeinen Formel I gegen tierische Schädlinge ließ sich durch folgende Versuche zeigen

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

Wirkung gegen Nephotettix cincticeps, Grüne Reiszikade (Kontaktversuch)

Rundfilter (⌀ 9 cm) wurden mit 1 cm³ der wäßrigen Wirkstoffaufarbeitung behandelt und in eine Kunststoffpetrischale mit Nocken (⌀ 94 mm) gelegt. Anschließend setzte man 5 adulte Reiszikaden ein und verschloß die Petrischale.

Nach 24 h wurde die Mortalität bonitiert.

In diesem Versuch zeigten die Verbindungen 8, 9 und 16 eine Wirkschwelle von 200 ppm.

## Patentansprüche

1. Phenylcarbamate der Formel I 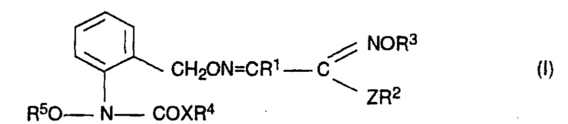 in der die Substituenten die folgende Bedeutung haben:
X eine direkte Bindung, O oder NR^{a};
R^{a} Wasserstoff oder Alkyl;
Z O, S oder NR^{b};
R^{b} Wasserstoff, C₁-C₆-Alkyl;, C₃-C₆-Alkenyl oder C₃-C₆-Alkinyl;
R¹ Wasserstoff, C₁-C₆-Alkyl oder C₁-C₄-Halogenalkyl;
R² C₂-C₆-Alkyl, C₃-C₆-Alkenyl oder C₃-C₆-Alkinyl, wobei diese Gruppen partiell oder vollständig halogeniert sein können und/oder einen bis drei der folgenden Reste tragen können:
- Cyano, Nitro, Amino, Hydroxy,
- C₁-C₆-Alkoxy, C₁-C₆-Halogenalkoxy, C₁-C₆-Alkylthio, C₁-C₆-Halogenalkylthio, C₁-C₆-Alkylsulfonyl, C₁-C₆-Alkylsulfoxyl, C₁-C₆-Alkylamino, Di-C₁-C₆-alkylamino,
- C₃-C₆-Cycloalkyl, Aryl, Hetaryl oder Oxiranyl, wobei die cyclischen Reste ihrerseits partiell oder vollständig halogeniert sein können und/oder eine bis drei der folgenden Gruppen tragen können: Cyano, Nitro, Amino, Aminocarbonyl, Aminothiocarbonyl, C₁-C₄-Alkyl, C₁-C₄-Halogenalkyl, C₁-C₄-Alkylcarbonyl, C₁-C₆-Alkoxy, C₁-C₆-Halogenalkoxy, C₁-C₆-Alkoxycarbonyl, C₁-C₆-Alkylthio, C₁-C₆-Halogenalkylthio, C₁-C₆-Alkylsulfonyl, C₁-C₆-Alkylsulfoxyl, C₁-C₆-Alkylamino, Di-C₁-C₆-alkylamino, C₁-C₆-Alkylaminocarbonyl, Di-C₁-C₆-alkylaminocarbonyl, C₂-C₆-Alkenyloxy und Phenyl;
Methyl, welches ein bis drei Halogenatome trägt und/oder einen der folgenden Reste trägt:
- Cyano, Nitro,
- C₁-C₆-Alkoxy, C₁-C₆-Halogenalkoxy, C₁-C₆-Alkylthio, C₁-C₆-Halogenalkylthio, C₁-C₆-Alkylsulfonyl, C₁-C₆-Alkylsulfoxyl, C₁-C₆-Alkylamino, Di-C₁-C₆-alkylamino,
- C₃-C₆-Cycloalkyl, Aryl, Hetaryl oder Oxiranyl, wobei die cyclischen Reste ihrerseits partiell oder vollständig halogeniert sein können und/oder eine bis drei der folgenden Gruppen tragen können: Cyano, Nitro, Amino, Aminocarbonyl, Aminothiocarbonyl, C₁-C₄-Alkyl, C₁-C₄-Halogenalkyl, C₁-C₄-Alkylcarbonyl, C₁-C₆-Alkoxy, C₁-C₆-Halogenalkoxy, C₁-C₆-Alkoxycarbonyl, C₁-C₆-Alkylthio, C₁-C₆-Halogenalkylthio, C₁-C₆-Alkylsulfonyl, C₁-C₆-Alkylsulfoxyl, C₁-C₆-Alkylamino, Di-C₁-C₆-alkylamino, C₁-C₆-Alkylaminocarbonyl, Di-C₁-C₆-alkylaminocarbonyl, C₂-C₆-Alkenyloxy und Phenyl;
C₃-C₆-Cycloalkyl, wobei diese Gruppen partiell oder vollständig halogeniert sein können und/oder einen bis drei der folgenden Reste tragen können:
- Cyano, Nitro, Amino, Hydroxy,
- C₁-C₄-Alkyl, C₁-C₄-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfonyl, C₁-C₆-Alkylsulfoxyl, C₁-C₆-Alkylamino, Di-C₁-C₆-alkylamino,
R³ C₁-C₆-Alkyl, C₂-C₆-Alkenyl oder C₂-C₆-Alkinyl, wobei diese Gruppen partiell oder vollständig halogeniert sein können und/oder einen bis drei der folgenden Reste tragen können:
- Cyano, C₁-C₃-Alkoxy,
- Oxiranyl oder C₃-C₆-Cycloalkyl, wobei die cyclischen Reste ihrerseits partiell oder vollständig halogeniert sein können und/oder eine bis drei C₁-C₄-Alkylgruppen tragen können;
R⁴ Alkyl, Alkenyl oder Alkinyl, und für den Fall, daß X für NR^{a} steht, zusätzlich Wasserstoff;
R⁵ Wasserstoff,
ggf. subst. Alkyl, Alkenyl, Alkinyl, Alkylcarbonyl oder Alkoxycarbonyl.

2. Verbindungen der Formel I gemäß Anspruch 1, in denen R² die folgenden Bedeutung hat:
C₂-C₆-Alkyl, C₃-C₆-Alkenyl oder C₃-C₆-Alkinyl, wobei diese Gruppen partiell oder vollständig halogeniert sein können und/oder einen bis drei der folgenden Reste tragen können:
- C₃-C₆-Cycloalkyl, Phenyl oder Oxiranyl, wobei die cyclischen Reste ihrerseits partiell oder vollständig halogeniert sein können und/oder eine bis drei der folgenden Gruppen tragen können: Cyano, C₁-C₄-Alkyl, C₁-C₄-Halogenalkyl, C₁-C₆-Alkoxy, C₁-C₆-Halogenalkoxy, C₁-C₆-Alkoxycarbonyl, C₁-C₆-Alkylthio, C₁-C₆-Halogenalkylthio und Phenyl;
Methyl, welches ein bis drei Halogenatome trägt und/oder einen der folgenden Reste trägt:
- Cyano, Nitro,
- C₃-C₆-Cycloalkyl, Aryl, Hetaryl oder Oxiranyl, wobei die cyclischen Reste ihrerseits partiell oder vollständig halogeniert sein können und/oder eine bis drei der folgenden Gruppen tragen können: Cyano, Nitro, Amino, Aminocarbonyl, Aminothiocarbonyl, C₁-C₄-Alkyl C₁-C₄-Halogenalkyl, C₁-C₄-Alkylcarbonyl, C₁-C₄-Alkoxy, C₁-C₄-Halogenalkoxy, C₁-C₄-Alkoxycarbonyl, C₁-C₄-Alkylthio, C₁-C₄-Halogenalkylthio, C₁-C₄-Alkylsulfonyl, C₁-C₄-Alkylsulfoxyl, C₁-C₄-Alkylamino, Di-C₁-C₄-alkylamino, C₁-C₄-Alkylaminocarbonyl, Di-C₁-C₄-alkylaminocarbonyl, C₂-C₄-Alkenyloxy und Phenyl;
C₃-C₆-Cycloalkyl, wobei diese Gruppen partiell oder vollständig halogeniert sein können und/oder einen bis drei der folgenden Reste tragen können:
- Cyano, C₁-C₄-Alkyl, C₁-C₄-Halogenalkyl, C₁-C₆-Alkoxy, C₁-C₆-Halogenalkoxy, C₁-C₆-Alkylthio, C₁-C₆-Halogenalkylthio.

3. Verbindungen der Formel I gemäß Anspruch 1, in denen Z für Sauerstoff oder Schwefel steht.

4. Verfahren zur Herstellung der Verbindungen der Formel I gemäß Anspruch 1, **dadurch gekennzeichnet**, daß man ein Benzylderivat der Formel II 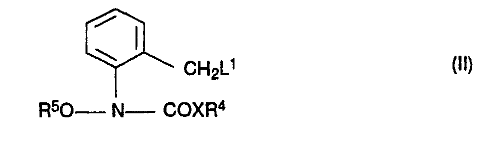 in der L¹ für eine nucleophil austauschbare Abgangsgruppe steht, in an sich bekannter Weise mit einem Hydroxyimin der Formel III 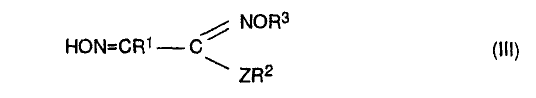 umsetzt.

5. Verfahren zur Herstellung der Verbindungen der Formel I gemäß Anspruch 1, **dadurch gekennzeichnet**, daß man ein Benzylderivat der Formel II gemäß Anspruch 4 in an sich bekannter Weise mit einem Dihydroxyimin der Formel IV 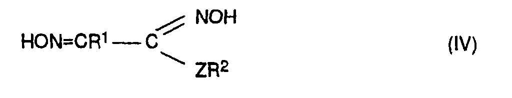 zu einer Verbindung der Formel V 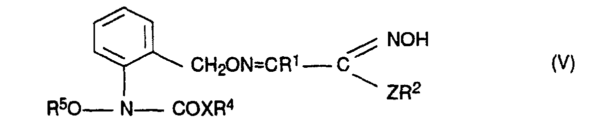 umsetzt und V anschließend mit einer Verbindung der Formel VIa
R³―L² (VIa)
in der L² für eine nucleophil austauschbare Abgangsgruppe steht, zu I umsetzt.

6. Verfahren zur Herstellung der Verbindungen I gemäß Anspruch 1, in denen R⁵ Wasserstoff bedeutet (I.a), **dadurch gekennzeichnet**, daß man ein Nitrobenzol der Formel VII 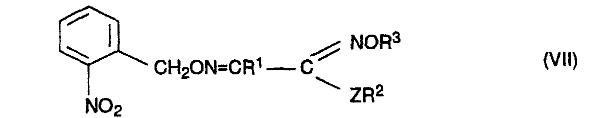 zum entsprechenden Hydroxylamin VIII 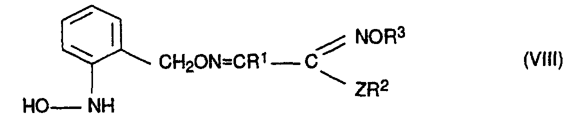 reduziert und VIII anschließend durch Umsetzung mit einem Acylierungsmittel der Formel VIb
L³―COXR⁴ (VIb)
in der L³ für Halogen oder Aryloxy steht, zu I.a umsetzt.

7. Verfahren zur Herstellung der Verbindungen I gemäß Anspruch 1, in denen R⁵ nicht Wasserstoff bedeutet (I.b), **dadurch gekennzeichnet**, daß man eine Verbindungen der Formel I.a gemäß Anspruch 6 in an sich bekannter Weise mit einer Verbindung der Formel VIc
R⁵―L⁴ (VIc)
in der L⁴ für Halogen, Mesylat, Tosylat, Carboxylat und Sulfat steht, umsetzt.

8. Zur Bekämpfung von tierischen Schädlingen oder Schadpilzen geeignetes Mittel, enthaltend einen festen oder flüssigen Trägerstoff und eine Verbindung der allgemeinen Formel I gemäß Anspruch 1.

9. Verwendung der Verbindungen I gemäß Anspruch 1 zur Herstellung eines zur Bekämpfung von tierischen Schädlingen oder Schadpilzen geeigneten Mittels.

10. Verfahren zur Bekämpfung von Schadpilzen, **dadurch gekennzeichnet**, daß man die Pilze oder die vor Pilzbefall zu schützenden Materialien, Pflanzen, Boden oder Saatgüter mit einer wirksamen Menge einer Verbindung der allgemeinen Formel I gemäß Anspruch 1 behandelt.

11. Verfahren zur Bekämpfung von tierischen Schädlingen, **dadurch gekennzeichnet**, daß man die Schädlingen oder die von ihnen zu schützenden Materialien, Pflanzen, Boden oder Saatgüter mit einer wirksamen Menge einer Verbindung der allgemeinen Formel I gemäß Anspruch 1 behandelt.

12. Verwendung der Verbindungen der Formel I gemäß Anspruch 1 zur Bekämpfung von tierischen Schädlingen oder Schadpilzen.

13. Verbindungen der Formel Xa 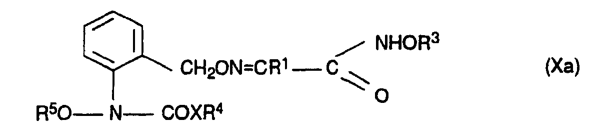 in der die Substituenten X, R¹, R³, R⁴ und R⁵ die in Anspruch 1 gegenbene Bedeutung haben.

14. Verwendung der Verbindungen Xa gemäß Anspruch 13 als Zwischenprodukte.

## Claims

1. A phenylcarbamate of the formula I 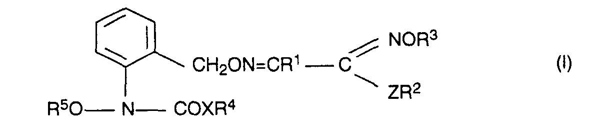 where
X is a direct bond, O or NR^{a};
R^{a} is hydrogen or alkyl;
Z is O, S or NR^{b};
R^{b} is hydrogen, C₁-C₆-alkyl, C₃-C₆-alkenyl or C₃-C₆-alkynyl;
R¹ is hydrogen, C₁-C₆-alkyl or C₁-C₄-haloalkyl; and
R² is C₂-C₆-alkyl, C₃-C₆-alkenyl or C₃-C₆-alkynyl, where these groups may be partially or completely halogenated or may carry from one to three of the following radicals:
- cyano, nitro, amino, hydroxyl,
- C₁-C₆-alkoxy, C₁-C₆-haloalkoxy, C₁-C₆-alkylthio, C₁-C₆-haloalkylthio, C₁-C₆-alkylsulfonyl, C₁-C₆-alkylsulfoxyl, C₁-C₆-alkylamino, di-C₁-C₆-alkylamino,
- C₃-C₆-cycloalkyl, aryl, hetaryl or oxiranyl, where the cyclic radicals in turn may be partially or completely halogenated or may carry from one to three of the following groups: cyano, nitro, amino, aminocarbonyl, aminothiocarbonyl, C₁-C₄-alkyl, C₁-C₄-haloalkyl, C₁-C₄-alkylcarbonyl, C₁-C₆-alkoxy, C₁-C₆-haloalkoxy, C₁-C₆-alkoxycarbonyl, C₁-C₆-alkylthio, C₁-C₆-haloalkylthio, C₁-C₆-alkylsulfonyl, C₁-C₆-alkylsulfoxyl, C₁-C₆-alkylamino, di-C₁-C₆-alkylamino, C₁-C₆-alkylaminocarbonyl, di-C₁-C₆-alkylaminocarbonyl, C₂-C₆-alkenyloxy and phenyl;
methyl, which carries from one to three halogen atoms or carries one of the following radicals:
- cyano, nitro,
- C₁-C₆-alkoxy, C₁-C₆-haloalkoxy, C₁-C₆-alkylthio, C₁-C₆-haloalkylthio, C₁-C₆-alkylsulfonyl, C₁-C₆-alkylsulfoxyl, C₁-C₆-alkylamino, di-C₁-C₆-alkylamino,
- C₃-C₆-cycloalkyl, aryl, hetaryl or oxiranyl, where the cyclic radicals in turn may be partially or completely halogenated or may carry from one to three of the following groups: cyano, nitro, amino, aminocarbonyl, aminothiocarbonyl, C₁-C₄-alkyl, C₁-C₄-haloalkyl, C₁-C₄-alkylcarbonyl, C₁-C₆-alkoxy, C₁-C₆-haloalkoxy, C₁-C₆-alkoxycarbonyl, C₁-C₆-alkylthio, C₁-C₆-haloalkylthio, C₁-C₆-alkylsulfonyl, C₁-C₆-alkylsulfoxyl, C₁-C₆-alkylamino, di-C₁-C₆-alkylamino, C₁-C₆-alkylaminocarbonyl, di-C₁-C₆-alkylaminocarbonyl, C₂-C₆-alkenyloxy and phenyl;
C₃-C₆-cycloalkyl, where these groups may be partially or completely halogenated or may carry from one to three of the following radicals:
- cyano, nitro, amino, hydroxyl,
- C₁-C₄-alkyl, C₁-C₄-haloalkyl, C₁-C₆-alkoxy, C₁-C₆-haloalkoxy, C₁-C₆-alkylthio, C₁-C₆-haloalkylthio, C₁-C₆-alkylsulfonyl, C₁-C₆-alkylsulfoxyl, C₁-C₆-alkylamino, di-C₁-C₆-alkylamino,
R³ is C₁-C₆-alkyl, C₂-C₆-alkenyl or C₂-C₆-alkynyl, where these groups may be partially or completely halogenated or may carry from one to three of the following radicals:
- cyano, C₁-C₃-alkoxy,
- oxiranyl or C₃-C₆-cycloalkyl, where the cyclic radicals in turn may be partially or completely halogenated or may carry from one to three C₁-C₄-alkyl groups; and
R⁴ is alkyl, alkenyl or alkynyl, and, where X is NR^{a}, additionally hydrogen; and
R⁵ is hydrogen or
unsubstituted or substituted alkyl, alkenyl, alkynyl, alkylcarbonyl or alkoxycarbonyl.

2. A compound of the formula I as claimed in claim 1, in which R² has the following meanings
C₂-C₆-alkyl, C₃-C₆-alkenyl or C₃-C₆-alkynyl, where these groups may be partially or completely halogenated or may carry from one to three of the following radicals:
- C₃-C₆-cycloalkyl, phenyl or oxiranyl, where the cyclic radicals in turn may be completely or partially halogenated or may carry from 1 to 3 of the following groups: cyano, C₁-C₄-alkyl, C₁-C₄-haloalkyl, C₁-C₆-alkoxy, C₁-C₆-haloalkoxy, C₁-C₆-alkoxycarbonyl, C₁-C₆-alkylthio, C₁-C₆-haloalkylthio and phenyl;
methyl, which carries from one to three halogen atoms or carries one of the following radicals:
- cyano, nitro,
- C₃-C₆-cycloalkyl, aryl, hetaryl or oxiranyl, where the cyclic radicals in turn may be partially or completely halogenated or may carry from one to three of the following groups: cyano, nitro, amino, aminocarbonyl, aminothiocarbonyl, C₁-C₄-alkyl, C₁-C₄-haloalkyl, C₁-C₄-alkylcarbonyl, C₁-C₄-alkoxy, C₁-C₄-haloalkoxy, C₁-C₄-alkoxycarbonyl, C₁-C₄-alkylthio, C₁-C₄-haloalkylthio, C₁-C₄-alkylsulfonyl, C₁-C₄-alkylsulfoxyl, C₁-C₄-alkylamino, di-C₁-C₄-alkylamino, C₁-C₄-alkylaminocarbonyl, di-C₁-C₄-alkylaminocarbonyl, C₂-C₄-alkenyloxy and phenyl;
C₃-C₆-cycloalkyl, where these groups may be partially or completely halogenated or may carry from one to three of the following radicals:
- cyano, C₁-C₄-alkyl, C₁-C₄-haloalkyl, C₁-C₆-alkoxy, C₁-C₆-haloalkoxy, C₁-C₆-alkylthio and C₁-C₆-haloalkylthio.

3. A compound of the formula I as claimed in claim 1, in which Z is oxygen or sulfur.

4. A process for the preparation of a compound of the formula I as claimed in claim 1, wherein a benzyl derivative of the formula II 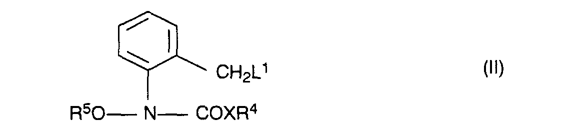 where L¹ is a nucleophilically substitutable leaving group, is reacted in a manner known per se with a hydroxyimine of the formula III

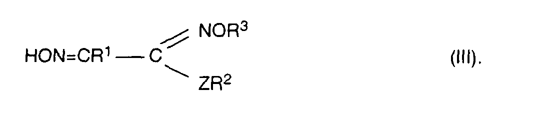

5. A process for the preparation of a compound of the formula I as claimed in claim 1, wherein a benzyl derivative of the formula II as claimed in claim 4 is reacted in a manner known per se with a dihydroxyimine of the formula IV 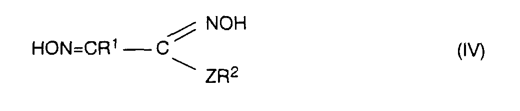 to give a compound of the formula V 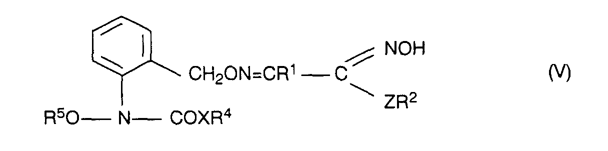 and V is then reacted with a compound of the formula VIa
R³―L² (VIa)
where L² is a nucleophilically substitutable leaving group, to give I.

6. A process for the preparation of a compound I as claimed in claim 1, in which R⁵ is hydrogen (I.a), wherein a nitrobenzene of the formula VII 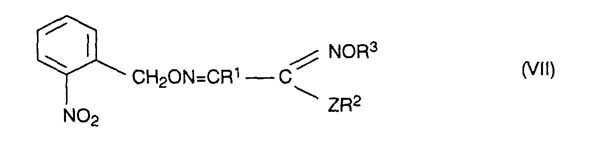 is reduced to the corresponding hydroxylamine VIII 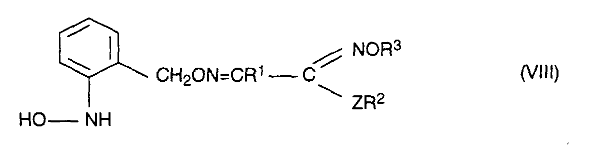 and VIII is then converted into I.a by reaction with an acylating agent of the formula VIb
L³―COXR⁴ (VIb)
where L³ is halogen or aryloxy.

7. A process for the preparation of a compound I as claimed in claim 1, in which R⁵ is not hydrogen (I.b), wherein a compound of the formula I.a as claimed in claim 6 is reacted in a manner known per se with a compound of the formula VIc
R⁵―L⁴ (VIc
where L⁴ is halogen, mesylate, tosylate, carboxylate or sulfate.

8. An agent suitable for controlling animal pests or harmful fungi, and containing a solid or liquid carrier and a compound of the formula I as claimed in claim 1.

9. Use of a compound I as claimed in claim 1 for the preparation of an agent suitable for controlling animal pests or harmful fungi.

10. A method for controlling harmful fungi, wherein the fungi or the materials, plants, soil or seeds to be protected from fungal attack are treated with an effective amount of a compound of the formula I as claimed in claim 1.

11. A method for controlling animal pests, wherein the pests or the materials, plants, soil or seeds to be protected from them are treated with an effective amount of a compound of the formula I as claimed in claim 1.

12. Use of a compound of the formula I as claimed in claim 1 for controlling animal pests or harmful fungi.

13. A compound of the formula Xa 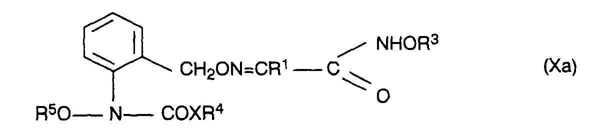 where X, R¹, R³, R⁴ and R⁵ have the meanings stated in claim 1.

14. Use of the compound Xa as claimed in claim 13 as an intermediate.

## Revendications

1. Carbamates de phényle de formule I 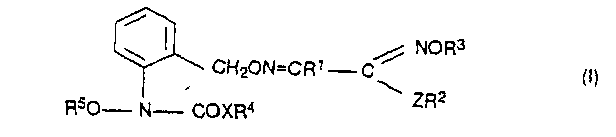 dans laquelle les substituants ont la signification suivante:
X une liaison directe, O ou NR^{a};
R^{a} hydrogène ou alkyle;
Z O, S ou NR^{b};
R^{b} hydrogène, alkyle en C₁-C₆, alcényle en C₃-C₆ ou alcynyle en C₃-C₆;
R¹ hydrogène, alkyle en C₁-C₆ ou halogénoalkyle en C₁-C₄;
R² alkyle en C₂-C₆, alcényle en C₃-C₆ ou alcynyle en C₃-C₆, tandis que ces groupes peuvent être partiellement ou totalement halogénés et/ou peuvent porter un à trois des restes suivants:
- cyano, nitro, amino, hydroxy,
- alcoxy en C₁-C₆, halogénoalcoxy en C₁-C₆, alkylthio en C₁-C₆, halogénoalkylthio en C₁-C₆, alkylsulfonyle en C₁-C₆, alkylsulfoxyle en C₁-C₆, alkylamino en C₁-C₆, di(alkyl en C₁-C₆)amino,
- cycloalkyle en C₃-C₆, aryle, hétaryle ou oxiranyle, tandis que les restes cycliques peuvent être à leur tour partiellement ou totalement halogénés et/ou peuvent porter un à trois des groupes suivants: cyano, nitro, amino, aminocarbonyle, aminothio-carbonyle, alkyle en C₁-C₄, halogénoalkyle en C₁-C₄, alkyl(C₁-C₄)carbonyle, alcoxy en C₁-C₆, halogénoalcoxy en C₁-C₆, alcoxy(C₁-C₆)carbonyle, alkylthio en C₁-C₆, halogénoalkylthio en C₁-C₆, alkylsulfonyle en C₁-C₆, alkylsulfoxyle en C₁-C₆, alkylamino en C₁-C₆, di(alkyl en C₁-C₆)-amino, alkyl(C₁-C₆)aminocarbonyle, di(alkyl en C₁-C₆)aminocarbonyle, alcényloxy en C₂-C₆ et phényle;
méthyle, qui porte un à trois atomes d'halogène et/ou l'un des restes suivants:
- cyano, nitro,
- alcoxy en C₁-C₆, halogénoalcoxy en C₁-C₆, alkylthio en C₁-C₆, halogénoalkylthio en C₁-C₆, alkylsulfonyle en C₁-C₆, alkyl-sulfoxyle en C₁-C₆, alkylamino en C₁-C₆, di(alkyl en C₁-C₆)amino,
- cycloalkyle en C₃-C₆, aryle, hétaryle ou oxiranyle, tandis que les restes cycliques peuvent être à leur tour partiellement ou totalement halogénés et/ou peuvent porter un à trois des groupes suivants: cyano, nitro, amino, aminocarbonyle, aminothiocarbonyle, alkyle en C₁-C₄, halogénoalkyle en C₁-C₄, alkyl(C₁-C₄)carbonyle, alcoxy en C₁-C₆, halogénoalcoxy en C₁-C₆, alcoxy(C₁-C₆)carbonyle, alkylthio en C₁-C₆, halogénoalkylthio en C₁-C₆, alkylsulfonyle en C₁-C₆, alkylsulfoxyle en C₁-C₆, alkylamino en C₁-C₆, di(alkyl en C₁-C₆)-amino, alkyl(C₁-C₆)aminocarbonyle, di(alkyl en C₁-C₆)-aminocarbonyle, alcényloxy en C₂-C₆ et phényle;
cycloalkyle en C₃-C₆, tandis que ces groupes peuvent être partiellement ou totalement halogénés et/ou peuvent porter un à trois des restes suivants:
- cyano, nitro, amino, hydroxy,
- alkyle en C₁-C₄, halogénoalkyle en C₁-C₄, alcoxy en C₁-C₆, halogénoalcoxy en C₁-C₆, alkylthio en C₁-C₆, halogénoalkylthio en C₁-C₆, alkylsulfonyle en C₁-C₆, alkyl-sulfoxyle en C₁-C₆, alkylamino en C₁-C₆, di(alkyl en C₁-C₆)amino,
R³ alkyle en C₁-C₆, alcényle en C₂-C₆ ou alcynyle en C₂-C₆, tandis que ces groupes peuvent être partiellement ou totalement halogénés et/ou peuvent porter un à trois des restes suivants:
- cyano, alcoxy en C₁-C₃,
- oxiranyle ou cycloalkyle en C₃-C₆, tandis que les restes cycliques peuvent à leur tour être partiellement ou totalement halogénés et/ou peuvent porter un à trois groupes alkyle en C₁-C₄;
R⁴ alkyle, alcényle ou alcynyle, et dans le cas où X désigne NRa, en outre l'hydrogène;
R⁵ hydrogène,
alkyle, alcényle, alcynyle, alkylcarbonyle ou alcoxycarbonyle, éventuellement substitués.

2. Composés de formule I selon la revendication 1, dans lesquels R² a la signification suivante:
alkyle en C₂-C₆, alcényle en C₃-C₆ ou alcynyle en C₃-C₆, tandis que ces groupes peuvent être partiellement ou totalement halogénés et/ou peuvent porter un à trois des groupes suivants:
- cycloalkyle en C₃-C₆, phényle ou oxiranyle, tandis que les restes cycliques peuvent à leur tour être partiellement ou totalement halogénés et/ou peuvent porter un à trois des groupes suivants: cyano, alkyle en C₁-C₄, halogénoalkyle en C₁-C₄, alcoxy en C₁-C₆, halogénoalcoxy en C₁-C₆, alcoxy(C₁-C₆)carbonyle, alkylthio en C₁-C₆, halogénoalkylthio en C₁-C₆ et phényle;
méthyle, qui porte un à trois atomes d'halogène et/ou l'un des restes suivants:
- cyano, nitro,
- cycloalkyle en C₃-C₆, aryle, hétaryle ou oxiranyle, tandis que les restes cycliques peuvent être à leur tour partiellement ou totalement halogénés et/ou peuvent porter un à trois des groupes suivants: cyano, nitro, amino, aminocarbonyle, aminothiocarbonyle, alkyle en C₁-C₄, halogénoalkyle en C₁-C₄, alkyl(C₁-C₄)carbonyle, alcoxy en C₁-C₄, halogénoalcoxy en C₁-C₄, alcoxy(C₁-C₄)carbonyle, alkylthio en C₁-C₄, halogénoalkylthio en C₁-C₄, alkylsulfonyle en C₁-C₄, alkylsulfoxyle en C₁-C₄, alkylamino en C₁-C₄, di(alkyl en C₁-C₄)-amino, alkyl(C₁-C₄)aminocarbonyle, di(alkyl en C₁-C₄)-aminocarbonyle, alcényloxy en C₂-C₄ et phényle;
cycloalkyle en C₃-C₆, tandis que ces groupes peuvent être partiellement ou totalement halogénés et/ou peuvent porter un à trois des restes suivants:
- cyano, alkyle en C₁-C₄, halogénoalkyle en C₁-C₄, alcoxy en C₁-C₆, halogénoalcoxy en C₁-C₆, alkylthio en C₁-C₆, halogénoalkylthio en C₁-C₆.

3. Composés de formule I selon la revendication 1, dans lesquels Z désigne l'oxygène ou le soufre.

4. Procédé pour la préparation des composés de formule I selon la revendication 1, caractérisés par le fait qu'on fait réagir un dérivé de benzyle de formule II 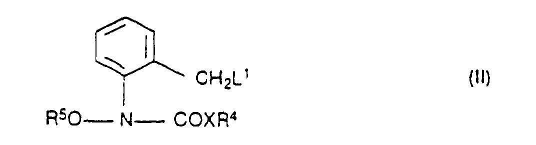 dans laquelle L¹ désigne un groupe éliminable échangeable par voie nucléophile, d'une manière connue en soi, avec une hydroxyimine de formule III

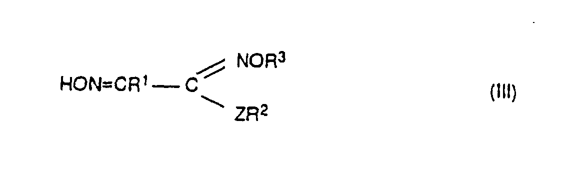

5. Procédé pour la préparation des composés de formule I selon la revendication 1, caractérisé par le fait qu'on fait réagir un dérivé de benzyle de formule II selon la revendication 4, d'une manière connue en soi, avec une dihydroxyimine de formule IV 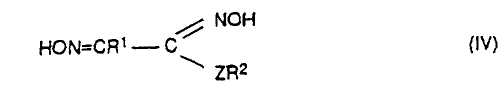 pour former un composé de formule V 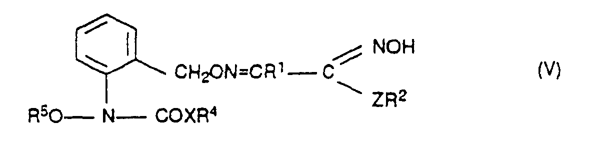 et on fait ensuite réagir V avec un composé de formule VIa
R³―L² (VIa)
dans laquelle L² désigne un groupe éliminable échangeable par voie nucléophile.

6. Procédé pour la préparation des composés I selon la revendication 1, dans lesquels R⁵ désigne l'hydrogène (I.a), caractérisé par le fait qu'on réduit un nitrobenzène de formule VII 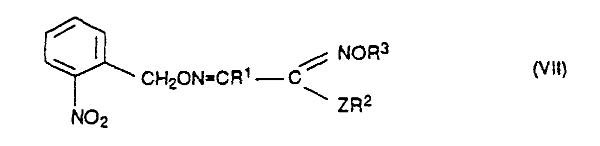 en l'hydroxylamine VIII correspondante 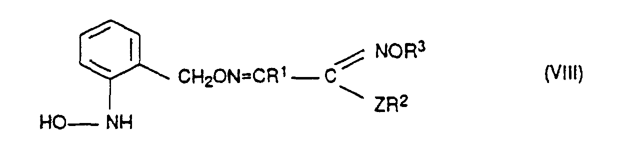 et on convertit ensuite VIII en I.a par réaction avec un agent d'acylation de formule VIb
L³― COXR⁴ (VIb)
dans laquelle L³ désigne un groupe halogéno ou aryloxy.

7. Procédé pour la préparation des composés I selon la revendication 1, dans lesquels R⁵ ne désigne pas l'hydrogène (I.b), caractérisé par le fait qu'on fait réagir un composé de formule I.a selon la revendication , d'une manière connue en soi, avec un composé de formule VIc
R⁵―L⁴ (VIc)
dans laquelle L⁴ désigne un groupe halogéno, mésylate, tosylate, carboxylate et sulfate.

8. Produit approprié pour la lutte contre les parasites animaux ou les champignons nuisibles, contenant un support solide ou fluide et un composé de formule I selon la revendication 1.

9. Utilisation des composés I selon la revendication 1 pour la préparation d'un produit approprié pour la lutte contre les parasites animaux ou les champignons nuisibles.

10. Procédé pour la lutte contre les champignons nuisibles, caractérisé par le fait qu'on traite les champignons ou les matériaux, plantes, sols ou semences à protéger contre l'infestation par des champignons avec une quantité efficace d'un composé de formule générale I selon la revendication 1.

11. Procédé pour la lutte contre les parasites animaux, caractérisé par le fait qu'on traite les parasites ou les matériaux, plantes, sols ou semences à protéger contre eux avec une quantité efficace d'un composé de formule générale I selon la revendication 1.

12. Utilisation des composés de formule I selon la revendication 1 pour la lutte contre les parasites animaux ou les champignons nuisibles.

13. Composés de formule Xa 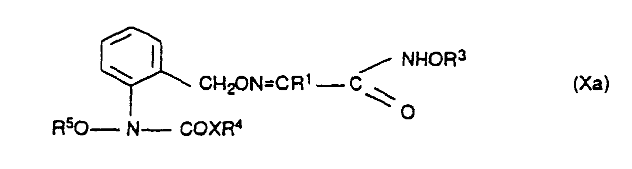 dans laquelle les substituants X, R¹, R³, R⁴ et R⁵ ont la signification indiquée dans la revendication 1.

14. Utilisation des composés Xa selon la revendication 13 comme produits intermédiaires.
